# EUROPEAN PATENT APPLICATION

(11) **EP 2 151 190 A1**
(43) Date of publication of application: **10.02.2010**
(21) Application number: 08161781.3
(22) Date of filing: 05.08.2008
(51) Int. Cl.: A61B 5/0428, H01R 13/66

(54) **Trace measuring connector**

(71) Applicant: Tseng Tsung-I, 302 Hsinchu County 30271 (TW)
(72) Inventor: Tseng Tsung-I, 302 Hsinchu County 30271 (TW)
(74) Representative: Lang, Christian

(57) **Abstract**

The present invention is a connector for measuring a trace physical quantity in a near-inductionless way. The present invention uses a scalable signal terminal with a proper volume ratio or a proper ratio of conductivity coefficient for accomplishing the near-inductionless measurement. Thus, such as, a microvolt of a liquid (e.g. blood, saliva, etc.) can be measured with the present invention.

## Description

### Field of the invention

The present invention relates to a measuring connector; more particularly, relates to measure a trace physical quantity in a near-inductionless way.

### Description of the Related Art

A common measuring device for blood comprises a body and a connector.

The body has a processor and related circuits inside for controlling operation and detecting blood density; and a signal hole and a socket outside for connecting to the circuits inside.

The connector comprises a base, a connecting wire and a joint. Therein, the base forms a socket for a detected object to be plugged in; an end of the connecting wire is set in the socket to be electrically connected with the detected object; and, another end of the connecting wire is connected with the joint to be plugged into the signal hole of the body for transmitting a signal of the detected object to the body. The joint is a USB joint to be plugged into the signal hole of the body with the same interface protocol. Or, the joint can be a phone jack to be plugged in the signal hole of the body. Accordingly, the measuring device for blood is used to measure blood-related material.

Although the general measuring device for blood measures blood-related material, the device is complex and the USB joint and the phone jack used for transmitting signal can not transmit trace signal measured in blood. Consequently, an inductive measuring is required. Hence, the prior art does not fulfill all users' requests on actual use.

### Summary of the invention

The main purpose of the present invention is to measure a microvolt of a liquid in a near-inductionless way.

To achieve the above purpose, the present invention is a trace measuring connector, comprising a shell and a transmitting unit, where the transmitting unit is combined with the shell; the transmitting unit comprises a first reference signal terminal, a second reference signal terminal and two scalable signal terminals; each terminal has two ends extended out of the shell; the scalable signal terminal has a volume ratio between 1:1.05 and 1:1000 or a ratio of conductivity coefficient between 1 :0.001 and 1 :0.995; the shell is covered with a shielding unit; and the shielding unit has a jack holding an end of each terminal. Accordingly, a novel trace measuring connector is obtained.

### Brief description of the drawings

The present invention will be better understood from the following detailed description of the preferred embodiment according to the present invention, taken in conjunction with the accompanying drawings, in which
- FIG.1: is a perspective view showing the preferred embodiment according to the present invention;
- FIG.2: is an explosive view showing the preferred embodiment;
- FIG.3: is a structural view showing the state of use; and
- FIG.4: is a flow view showing the state of use.

### Description of the preferred embodiment

The following description of the preferred embodiment is provided to understand the features and the structures of the present invention.

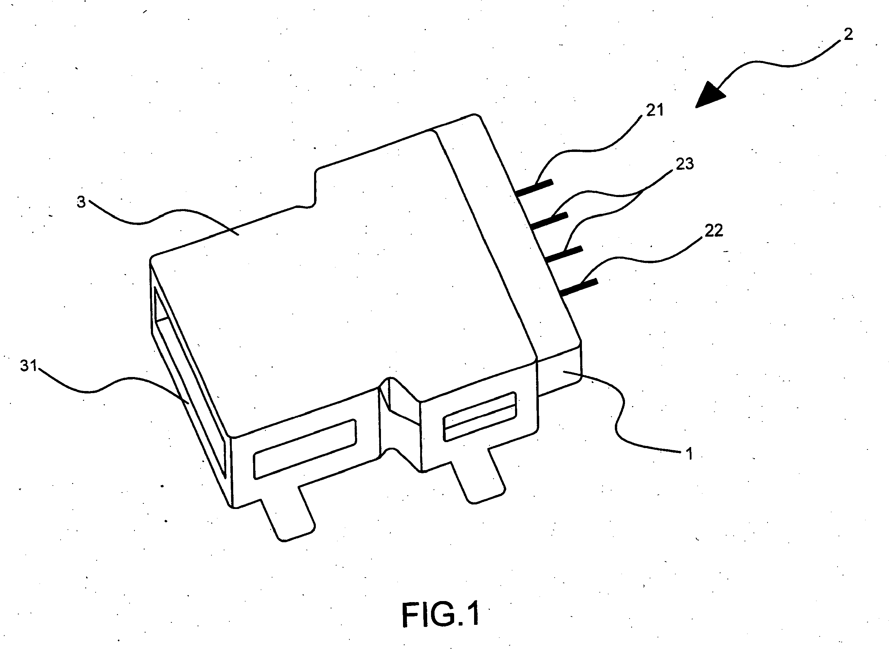

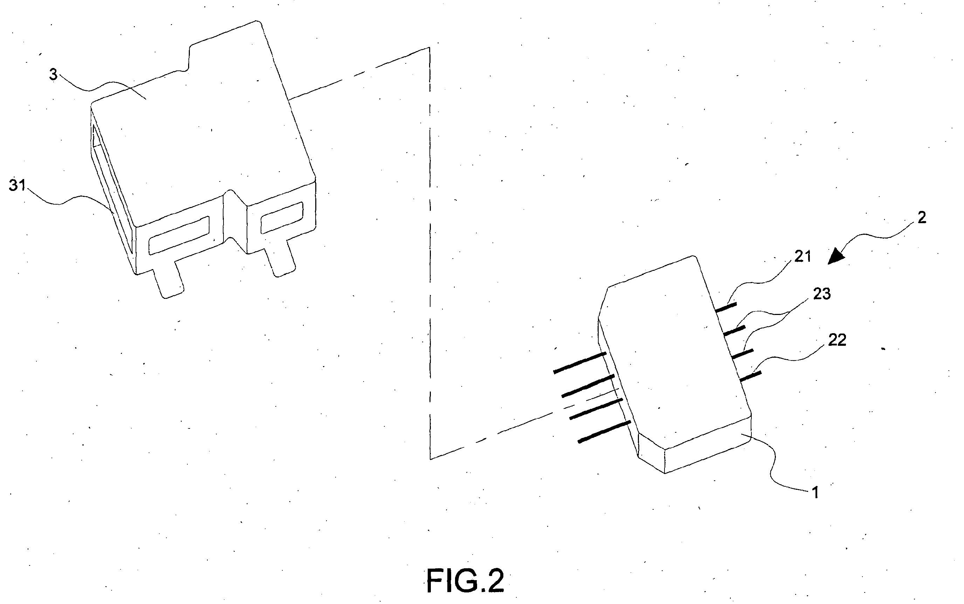

Please refer to FIG.1 and FIG.2, which are a perspective view and an explosive view showing a preferred embodiment according to the present invention. As shown in the figures, the present invention is a trace measuring connector, comprising a shell 1, a transmitting unit 2 and a shielding unit 3, where the present invention is used to measure microvolt of a liquid in a near-inductionless way.

The shell 1 is made of a plastic material.

The transmitting unit 2 is combined with the shell 1, where the shell 1 is combined at outside of the transmitting unit 2 through a jack-in-type injection molding or an insert molding. The transmitting unit 2 comprises a first reference signal terminal 21, a second reference signal terminal 22 and two scalable signal terminals 23. Each terminal 21,22,23 has two ends extended out of the shell 1; and the scalable signal terminal 23 has a volume ratio between 1:1.05 and 1:1000 or a ratio of conductivity coefficient between 1:0.001 and 1 :0.995. The scalable signal terminal 23 is made of copper, phosphor bronze, beryllium copper or any low-impedance copper covered with gold membrane. The first reference signal terminal 21 is a grounded terminal; and, the second reference signal terminal 22 is a source terminal.

The shielding unit 3, made of a metal, is covered at outside of the shell 1. The shielding unit 3 has a jack, where the jack is made of a plastic material and holds a first end of each of the first reference signal terminal 21, the second reference signal terminal 22 and the scalable signal terminals 23. Thus, with the above structure, a novel trace measuring connector is obtained.

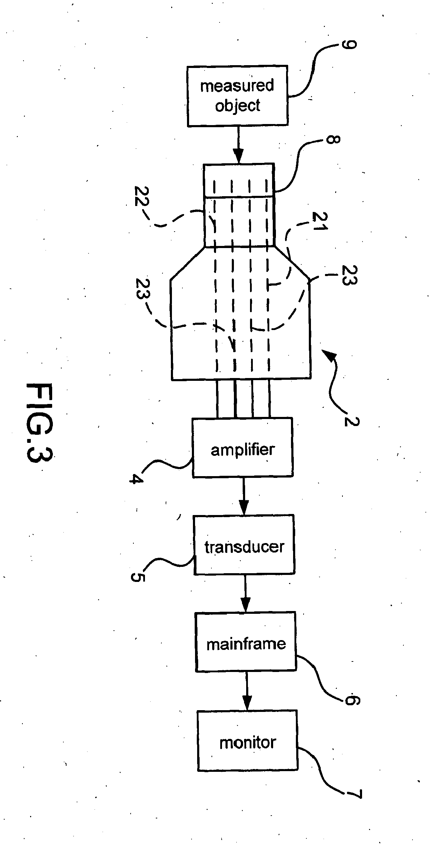

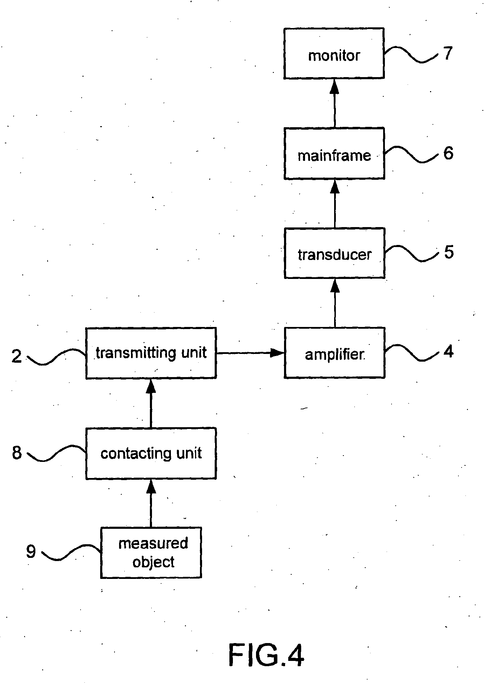

Please refer to FIG.3 and FIG.4, which are a structural view and a flow view showing a state of use. As shown in the figures, a transmitting unit 2 is assembled to a detecting device. On assembling to the detecting device, a second end of each of a first reference signal terminal 21, a second reference signal terminal 22 and two scalable signal terminals 23 is connected with an amplifier 4. The amplifier 4 is connected with a transducer 5. The transducer 5 is connected with a mainframe 6. And, the mainframe 6 is connected with a monitor 7. Therein, the transducer 5 is an analog/digital transducer; and, the monitor 7 is a charge-coupled device or a liquid-crystal display.

On using the present invention, a first end of each of the first reference signal terminal 21, the second reference signal terminal 22 and the scalable signal terminals 23 is connected with a contacting unit 8 (like a probe or wire). Then, the contacting unit 8 is contacted with a measured object 9, where the measured object 9 is a liquid having a microvolt (like blood, saliva, organic liquid or non-organic liquid). The operation of contacting is done in an invasive or non-invasive way and a near-inductionless way. After the contacting unit 8 is contacted with the measured object 9, a measured signal obtained along the contacting is amplified by the amplifier 4. Then, the signal of analog is transmitted to the transducer 5 by the transmitting unit 2 to be transformed into a signal of digital by the transducer 5. Finally, the digital signal is transmitted to the mainframe 6 for further related operation. Thus, a near-inductionless signal of 10⁻³ watt (W) to 10⁻¹⁶W is transformed into an inductive signal of 10⁰W to 10⁻⁷W for measuring a trace signal of 10⁻⁶W. In the end, a related resistance or conductance is shown on the monitor 7 for further operation.

To sum up, the present invention is a trace measuring connector, where a microvolt of a liquid is measured in a near-inductionless way.

The preferred embodiment herein disclosed is not intended to unnecessarily limit the scope of the invention. Therefore, simple modifications or variations belonging to the equivalent of the scope of the claims and the instructions disclosed herein for a patent are all within the scope of the present invention.

## Claims

1. A trace measuring connector, comprising
a shell; and
a transmitting unit,
wherein said transmitting unit is combined with said shell;
wherein said transmitting unit comprises a first reference signal terminal, a second reference signal terminal and two scalable signal terminals;
wherein each of said first reference signal terminal, said second reference signal terminal and said scalable signal terminals has two ends extended out of said shell; and
wherein said scalable signal terminal has a volume ratio between 1:1.05 and 1:1000.

2. The connector according to claim 1,
wherein said scalable signal terminal has a ratio of conductivity coefficient between 1:0.001 and 1 :0.995.

3. The connector according to claim 1,
wherein said shell is covered with a shielding unit; and
wherein said shielding unit has a jack holding a first end of each of said first reference signal terminal, said second reference signal terminal and said scalable signal terminals.

4. The connector according to claim 1,
wherein said shell is made of a plastic material.

5. The connector according to claim 1,
wherein said shell is combined at outside of said transmitting unit through a jack-in-type injection molding.

6. The connector according to claim 1,
wherein said shell is combined at outside of said transmitting unit through an insert molding.

7. The connector according to claim 1,
wherein a second end of each of said first reference signal terminal, said second reference signal terminal and said scalable signal terminals is connected with an amplifier;
wherein said amplifier is connected with a transducer;
wherein said transducer is connected with a mainframe; and
wherein said mainframe is connected with a monitor.

8. The connector according to claim 1,
wherein said transducer is an analog/digital transducer.

9. The connector according to claim 1,
wherein said monitor is a charge-coupled device.

10. The connector according to claim 1,
wherein said monitor is a liquid-crystal display.

11. The connector according to claim 1,
wherein said scalable signal terminal is made of copper.

12. The connector according to claim 1,
wherein said scalable signal terminal is made of phosphor bronze.

13. The connector according to claim 1,
wherein said scalable signal terminal is made of beryllium copper.

14. The connector according to claim 1,
wherein said scalable signal terminal is made of a low-impedance copper covered with gold membrane.

15. The connector according to claim 1,
wherein said shielding unit is made of a metal.

16. The connector according to claim 1,
wherein said jack is made of a plastic material.
